# EUROPÄISCHE PATENTANMELDUNG

(11) **EP 0 922 754 A1**
(43) Veröffentlichungstag der Anmeldung: **16.06.1999**
(21) Anmeldenummer: 98122341.5
(22) Anmeldetag: 25.11.1998
(51) Int. Cl.: C11D 3/20, C11D 1/37, A61K 7/50, A61K 7/48, C11D 1/29, C11D 1/12, C11D 1/04, C11D 3/04

(54) **Tensidhaltige Formulierung mit Perlglanzeffekt**

(30) Priorität: 10.12.1997 DE 19754842
(71) Anmelder: Clariant GmbH, 65929 Frankfurt am Main (DE)
(72) Erfinder: Turowski-Wanke, Angelika Dr., 65779 Kelkheim (DE); Naumann, Peter Dr., 65232 Taunusstein (DE)

(57) **Zusammenfassung**

Die Erfindung betrifft tensidhaltige Formulierungen mit Perlglanzeffekt in Form von Perlglanzkonzentraten oder gebrauchsfertigen perlglänzenden Formulierungen, die eine Fettsäure als perlglanzgebende Komponente, ein Alkylethersulfat oder Acylisethionat, Wasser und gegebenenfalls weitere Tenside und übliche Inhaltsstoffe enthalten.

Zur Verbesserung der thermischen Stabilität enthalten diese Formulierungen zusätzlich ein Zn-, Mg-, Cu-, Al- oder Ti-Salz.

## Beschreibung

Um Tensidformulierungen ein besseres Aussehen und damit auch einen höheren Handelswert zu geben, werden oft Perlglanzdispersionen eingearbeitet. Beispiele hierfür sind flüssige Wasch- und Reinigungsmittel (z.B. Bodenreiniger, Geschirrspülmittel) sowie flüssige kosmetische Präparate (z.B. Körperpflege- und Körperreinigungsmittel, Shampoos, Badezusätze, usw.). Perlglanzdispersionen verleihen Formulierungen einen seiden- bzw. perlmuttartiges Aussehen. Der Effekt entsteht durch Lichtstreuung an den dispergierten, meist blättchenförmigen Kristallen der perlglanzgebenden Komponenten.

Perlglanzdispersionen nach dem Stand der Technik bestehen hauptsächlich aus mindestens einer perlglanzgebenden Verbindung, mindestens einem Dispergiermittel und Wasser. Perlglanzgebende Verbindungen sind beispielsweise Fettsäuremonoalkanolamide, Fettsäuredialkanolamide, Monoester oder Diester von Ethylenglykol oder Mischungen davon, Propylenglykol oder dessen Oligomere, Mono- oder Diester von Alkylenglykolen mit Fettsäuren, Fettsäuren und deren Metallsalze, Monoester oder Polyester von Glycerin mit Carbonsäuren und Ketosulfone verschiedener Art.

In der letzten Zeit haben insbesondere freie Fettsäuren, wie etwa Behensäure verstärktes Interesse als perlglanzgebende Verbindung gefunden, Perlglanzdispersionen mit Fettsäuren sind beispielsweise beschrieben in DE-A-37 24 547 und DE-A-41 03 55. Neben der perlglanzgebenden Komponente und Wasser enthalten diese Perlglanzdispersionen noch ein oder mehrere Dispergiermittel und eventuell noch mehrwertige Alkohole, um die perlglanzgebende Verbindung in dispergierter Form zu halten. Problematisch bei diesen Perlglanzdispersionen ist die Tatsache, daß die Fettsäuren als perlglanzgebende Komponente bei höheren Temperaturen sich ganz oder teilweise auflösen, wodurch der Perlglanzeffekt verloren geht. Gleiches gilt auch für tensidhaltige Endformulierungen, die solche Perlglanzdispersionen enthalten.

Es wurde nun gefunden, daß sich die Temperaturbeständigkeit von tensidhaltigen Formulierungen mit Perlglanzeffekt auf Basis von Fettsäuren verbessern läßt, wenn man den tensidhaltigen Formulierungen ein im folgenden näher definiertes Metallsalz zugibt.

Tensidhaltige kosmetische Reinigungsmittel, die unter anderem wasserunlösliche Fettsäuresalze wie beispielsweise Zinkstearat, enthalten, sind aus GB-A-2 297 975 bekannt. Das Zinkstearat soll dabei ein verbessertes Hautgefühl vermitteln. Ebenso sind milde Hautreinigungsmittel mit einem Gehalt an wasserunlöslichen Fettsäuresalzen in US-A-5 607 678 beschrieben. In diesen Formulierungen sollen die Fettsäuresalze zusammen mit Fettalkoholen für die notwendige Viskosität sorgen und das Schaumvermögen verstärken.

Gegenstand der Erfindung sind tensidhaltige Formulierungen mit Perlglanzeffekt, die mindestens eine C₁₄-C₂₂-Fettsäure, ein Zn-, Mg-, Cu-, Al- oder Ti-Salz, ein Alkylethersulfat der Formel

R¹-O-(C₂H₄O)ₙ-SO₃^{⊖}X⁺

worin
- R¹: C₁₂- bis C₁₈-Alkyl,
- n: eine Zahl von 2 bis 8,
- X⁺: ein Alkalimetallion oder ein Ammoniumion bedeuten, und/oder
ein Acylisethionat der Formel

R²-CO-CH₂-CH₂-SO₃^{⊖}X⁺

worin
- R²: C₈- bis C₁₈-Alkyl,
- X⁺: ein Alkalimetallion oder ein Ammoniumion bedeuten,
sowie Wasser und gegebenenfalls weitere Tenside und übliche Inhaltsstoffe enthalten.

Bei den erfindungsgemäßen tensidhaltigen Formulierungen handelt es sich zum einen um Perlglanzdispersionen oder Perlglanzkonzentrate, die einen erhöhten Anteil an perlglanzgebender Verbindung enthalten. Zum anderen handelt es sich um übliche gebrauchsfertige tensidhaltige Formulierungen, denen zur Erreichung des Perlglanzeffekts ein solches Perlglanzkonzentrat zugesetzt wurde.

Die Perlglanzkonzentrate enthalten im allgemeinen 5 bis 30, vorzugsweise 10 bis 20 Gew.-% an Fettsäure, vorzugsweise Behensäure, 1 bis 50, vorzugsweise 1 bis 20 Gew.-% eines Alkylethersulfats oder Acylisethionats als Dispergiermittel oder deren Mischungen wie oben angegeben, 0,1 bis 20, vorzugsweise 1 bis 10 Gew.-% eines oder mehrerer der angegebenen Salze sowie Wasser und gegebenenfalls weitere übliche Inhaltsstoffe ad 100 Gew.-%. Als übliche Inhaltsstoffe können die Perlglanzkonzentrate beispielsweise bis zu 10 Gew.-% an mehrwertigen C₂- bis C₈-Alkoholen enthalten. Als mehrwertige Alkohole sind C₂- bis C₆-Polyole bevorzugt, insbesondere Ethylenglykol, 1,2- und 1,3-Propylenglykol, Glycerin, Di- und Triethylenglykol, Erythrit, Arabit, Adonit, Xylit, Sorbit, Mannit und Dulcit. Weitere übliche Inhaltsstoffe sind auch Konservierungsmittel und Puffersubstanzen. Bei den Zn-, Mg-, Cu-, Al- oder Ti-Salzen handelt es sich vorzugsweise um Chloride, Acetate oder andere wasserlösliche Salze dieser Kationen.

Die Herstellung der erfindungsgemäßen Perlglanzkonzentrate erfolgt bevorzugt in der Weise, daß Wasser, Metallsalz und Dispergiermittel zunächst in einem Mischgefäß (heizbarer Kessel) auf eine Temperatur von etwa 70 bis etwa 90°C erwärmt werden. Getrennt von diesem Vorgang wird die Fettsäure in einem zweiten Kessel bei einer Temperatur von im allgemeinen etwa 70 bis etwa 90°C homogen aufgeschmolzen. Die homogen aufgeschmolzene Fettsäure wird bei der angegebenen Temperatur von etwa 70 bis etwa 90°C unter Rühren in die Mischung aus Wasser, Metallsalz und Dispergiermittel eingebracht. Die erhaltene Dispersion wird zur Temperierung und Homogenisierung bei einer Temperatur von etwa 70 bis etwa 90°C unter Rühren gehalten (etwa 15 bis 30 Minuten). Unter Weiterrühren wird die Dispersion schrittweise auf eine Temperatur von 25°C abgekühlt. Die Viskosität des Perlglanzkonzentrats ist meistens so niedrig, daß auf den Einsatz besonderer Rühraggregate wie Homogenisatoren oder andere hochtourige Mischvorrichtungen verzichtet werden kann. Während des Abkühlens kristallisiert die Fettsäure aus, bei 25°C kann das dünnflüssige Perlglanz-Konzentrat abgefüllt werden.

Die erfindungsgemäßen Perlglanzkonzentrate eigenen sich zur Herstellung gebrauchsfertiger perlglänzender flüssiger, wäßriger Tensidformulierungen. Sie können beispielsweise in flüssige Feinwaschmittel, Universalwaschmittel, Handgeschirrspülmittel, Klarspüler, flüssige Reinigungs- und Desinfektionsmittel, flüssige Seifen, Haarshampoos, Haarspülungen, Haarfärbemittel, Haarwellmittel, Schaumbäder, Gesichtsreinigungsmittel, Duschbadzubereitungen und 2 in 1-Formulierungen eingearbeitet werden. Die erfindungsgemäßen Perlglanzkonzentrate unterstützen den Konditionierungseffekt von Haarshampoos, insbesondere von sogenannten 2 in 1 Shampoos, die wasserlösliche und/oder nicht wasserlösliche dispergierte Agenzien enthalten, wie etwa Siliconderivate (z.B. Dimethicone).

Die erfindungsgemäßen wäßrigen Perlglanzkonzentrate lassen sich in Tensidformulierungen leicht kalt einarbeiten, wodurch diese den angestrebten Perlglanz erhalten. Die erforderliche Menge an Perlglanzkonzentrat liegt bei 1 bis 10 Gew.-%, vorzugsweise 2 bis 5 Gew.-%, bezogen auf das Gewicht der zu behandelnden Tensidformulierung. Das Perlglanzkonzentrat wird in der angegebenen Menge den Tensidformulierungen vorzugsweise als letzte Komponente bei Raumtemperatur unter Rühren zugesetzt. Das Perlglanzkonzentrat entfaltet seinen Glanz und seine Ergiebigkeit in der Formulierung nach ca. 1 - 5 Tagen. Danach werden Endprodukte erhalten, die einen ausgezeichneten stabilen Perlglanz aufweisen.

Alternativ zu dieser Arbeitsweise kann man auch so vorgehen, daß man das Metallsalz erst bei der Herstellung der gebrauchsfertigen Tensidformulierung zugibt. Hierbei geht man von einem Perlglanzkonzentrat der oben beschriebenen Zusammensetzung aus, das wie zuvor angegeben hergestellt wurde, jedoch ohne Zusatz eines Metallsalzes. Dieses Perlglanzkonzentrat wird dann in die gebrauchsfertige Tensidformulierung eingearbeitet, zuvor wird jedoch das Metallsalz in Substanz oder als wäßrige Lösung zugegeben.

Die in den erfindungsgemäßen gebrauchsfertigen tensidhaltigen Formulierungen enthaltenen weiteren Tenside können anionische, nicht-ionische, kationische oder amphotere Tenside sein.

Als anionische Tenside kommen Sulfonate, Sulfate, Carboxylate, Phosphate und Mischungen aus den genannten Verbindungen in Betracht. Geeignete Kationen sind hierbei Alkalimetalle, wie z.B. Natrium oder Kalium oder Erdalkalimetalle, wie z.B. Calcium oder Magnesium sowie Ammonium, substituierte Ammoniumverbindungen, einschließlich Mono-, Di- oder Triethanolammoniumkationen und Mischungen der Kationen. Folgende Typen von anionischen Tensiden sind von besonderem Interesse: Alkylestersulfonate, Alkylsulfate, Alkylethersulfate, Alkylbenzolsulfonate, sekundäre Alkansulfonate, Seifen wie im folgenden beschrieben.

Alkylestersulfonate sind unter anderem lineare Ester von C₈-C₂₀-Carboxylsäuren(d.h. Fettsäuren), welche mittels gasförmigem SO₃ sulfoniert werden, wie in "The Journal of the American Oil Chemists Society" 52 (1975), pp. 323-329 beschrieben wird. Geeignete Ausgangsmaterialien sind natürliche Fette wie z.B. Tag, Palmöl oder Cocosöl, können aber auch synthetischer Natur sein. Bevorzugte Alkylestersulfonate, speziell für Waschmittelanwendungen, sind Verbindungen der Formel 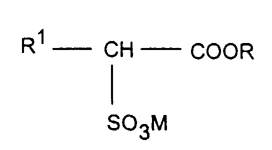 worin R¹ einen C₈-C₂₀-Kohlenwasserstoffrest, bevorzugt Alkyl und R einen C₁-C₆-Kohlenwasserstoffrest, bevorzugt Alkyl, darstellt. M steht für ein Kation, das ein wasserlösliches Salz mit dem Alkylestersulfonat bildet. Geeignete Kationen sind Natrium, Kalium, Lithium oder Ammoniumkationen, wie Monoethanolamin, Diethanolamin und Triethanolamin. Bevorzugt bedeuten R¹ C₁₀-C₁₆-Alkyl und R Methyl, Ethyl oder Isopropyl. Besonders bevorzugt sind Methylestersulfonate, in denen R¹ C₁₀-C₁₆-Alkyl bedeutet.

Alkylsulfate sind hier wasserlösliche Salze oder Säuren der Formel ROSO₃M, worin R bevorzugt ein C₁₀-C₂₄-Kohlenwasserstoffrest, bevorzugt C₁₀-C₂₀-Alkyl oder Hydroxyalkyl, besonders bevorzugt C₁₂-C₁₈-Alkyl- oder Hydroxyalkyl darstellt. M ist Wasserstoff oder ein Kation, z.B. ein Alkalimetallkation (z.B. Natrium, Kalium, Lithium), Ammonium oder substituiertes Ammonium z.B. Methyl-, Dimethyl- und Trimethylammoniumkationen und quaternäre Ammoniumkationen, wie Tetramethylammonium- und Dimethylpiperidiniumkationen und quaternäre Ammoniumkationen, abgeleitet von Alkylaminen wie Ethylamin, Diethylamin, Triethylamin und Mischungen davon. Alkylketten mit C₁₂-C₁₆ sind für niedrige Waschtemperaturen (z.B. unter ca. 50°C) und Alkylketten mit C₁₆-C₁₈ für höhere Waschtemperaturen (z.B. oberhalb ca. 50°C) bevorzugt.

Alkylethersulfate sind wasserlösliche Salze oder Säuren der Formel RO(A)ₘ SO₃M, worin R einen unsubstituierten C₁₀-C₂₄-Alkyl- oder Hydroxyalkylrest, bevorzugt einen C₁₂-C₂₀ Alkyl- oder Hydroxyalkylrest, besonders bevorzugt C₁₂-C₁₈-Alkyl oder Hydroxyalkylrest darstellt. A ist eine Ethoxy- oder Propoxyeinheit, m ist eine Zahl größer als 0, vorzugsweise zwischen ca. 0,5 und ca. 6, besonders bevorzugt zwischen ca. 0,5 und ca. 3 und M ist ein Wasserstoffatom oder ein Kation wie z.B. Natrium, Kalium, Lithium, Calcium, Magnesium, Ammonium oder ein substituiertes Ammoniumkation. Spezifische Beispiele von substituierten Ammoniumkationen sind Methyl-, Dimethyl-, Trimethylammonium- und quaternäre Ammoniumkationen wie Tetramethylammonium und Dimethylpiperidiniumkationen, sowie solche, die von Alkylaminen, wie Ethylamin, Diethylamin, Triethylamin, Mischungen davon abgeleitet sind. Als Beispiele seien C₁₂-C₁₈-Fettalkoholethersulfate genannt, wobei der Gehalt an Ethylenoxid 1, 2, 2.5, 3 oder 4 mol pro mol Fettalkoholethersulfat beträgt, und in denen M Natrium oder Kalium ist.

In sekundären Alkansulfonaten kann die Alkylgruppe entweder gesättigt oder ungesättigt, verzweigt oder linear und gegebenenfalls mit einer Hydroxylgruppe substituiert sein. Die Sulfogruppe kann eine beliebige Position an der gesamten C-Kette einnehmen, wobei die primären Methylgruppen am Kettenanfang und Kettenende keine Sulfogruppen besitzen. Die bevorzugten sekundären Alkansulfonate enthalten lineare Alkylketten mit ca. 9 bis 25 Kohlenstoffatomen, bevorzugt von ca. 1 0 bis ca. 20 Kohlenstoffatome und besonders bevorzugt ca. 13 bis 17 Kohlenstoffatome. Als Kation ist beispielsweise Natrium, Kalium, Ammonium, Mono-, Di- oder Triethanolammonium, Calcium oder Magnesium bevorzugt.

Weitere geeignete anionische Tenside sind Alkenyl- oder Alkylbenzolsulfonate. Die Alkenyl- oder Alkylgruppe kann verzweigt oder linear und gegebenenfalls mit einer Hydroxylgruppe substituiert sein. Die bevorzugten Alkylbenzolsulfonate enthalten lineare Alkylketten mit ca. 9 bis 25 Kohlenstoffatomen, bevorzugt von ca. 10 bis ca. 13 Kohlenstoffatome, das Kation ist Natrium, Kalium, Ammonium, Mono-, Di- oder Triethanolammonium, Calcium oder Magnesium und Mischungen davon. Für milde Tensidsysteme ist Magnesium als Kation bevorzugt, für Standardwaschanwendungen dagegen Natrium. Gleiches gilt für Alkenylbenzolsulfonate

Der Begriff anionische Tenside schließt auch Olefinsulfonate mit ein, die durch Sulfonierung von C₁₂-C₂₄-, vorzugsweise C₁₄-C₁₆-α-Olefinen mit Schwefeltrioxid und anschließende Neutralisation erhalten werden. Bedingt durch das Herstellverfahren, können diese Olefinsulfonate kleinere Mengen an Hydroxyalkansulfonaten und Alkandisulfonaten enthalten. Spezielle Mischungen von α-Olefinsulfonaten sind in US-3,332,880 beschrieben.

Weitere bevorzugte anionische Tenside sind Carboxylate, z.B. Fettsäureseifen und vergleichbare Tenside. Die Seifen können gesättigt oder ungesättigt sein und können verschiedene Substituenten, wie Hydroxylgruppen oder α-Sulfonatgruppen enthalten. Bevorzugt sind lineare gesättigte oder ungesättigte Kohlenwasserstoffreste mit ca. 6 bis ca. 30, bevorzugt ca. 10 bis ca. 18 Kohlenstoffatomen.
Als anionische Tenside kommen weiterhin Salze von Acylaminocarbonsäuren in Frage, die durch Umsetzung von Fettsäurechloriden mit Natriumsarkosinat erhalten werden, sowie Fettsäure-Eiweiß-Kondensationsprodukte, die durch Umsetzung von Fettsäurechloriden mit Oligopeptiden erhalten werden; Salze von Alkylsulfamidocarbonsäuren, Salze von Alkyl- und Alkylarylethercarbonsäuren-, C₈-C₂₄-Olefinsulfonate, sulfonierte Polycarboxylsäuren, hergestellt durch Sulfonierung der Pyrolyseprodukte von Erdalkalimetalicitraten, wie z.B. beschrieben in GB-1,082,179; Alkylglycerinsulfate, Fettacylglycerinsulfate, Alkylphenolethersulfate, primäre Paraffinsulfonate, Alkylphosphate, Alkyletherphosphate, Isethionate, wie Acylisethionate, N-Acyltauride, Alkylsuccinate, Sulfosuccinate, Monoester der Sulfosuccinate - (besonders gesättigte und ungesättigte C₁₂-C₁₈-Monoester) und Diester der Sulfosuccinate (besonders gesättigte und ungesättigte C₁₂-C₁₈-Diester), Acylsarcosinate, Sulfate von Alkylpolysacchariden wie Sulfate von Alkylpolyglycosiden, verzweigte primäre Alkylsulfate und Alkylpolyethoxycarboxylate wie die der Formel RO(CH₂CH₂)ₖCH₂COO⁻M⁺, worin R C₈-C₂₂-Alkyl, k eine Zahl von 0 bis 1 0 und M ein Kation ist, Harzsäuren oder hydrierte Harzsäuren, wie Rosin oder hydriertes Rosin oder Tallölharze und Tallölharzsäuren. Weitere Beispiele sind in "Surface Active Agents and Detergents" (Vol. 1 und 11, Schwartz, Perry und Berch) beschrieben.

Als nicht-ionische Tenside kommen beispielsweise folgende Typen in Frage:
Polyethylen-, Polypropylen- und Polybutylenoxidkondensate von Alkylphenolen.

Diese Verbindungen umfassen die Kondensationsprodukte von Alkylphenolen mit einer C₆-C₂₀-Alkylgruppe, die entweder linear oder verzweigt sein kann, mit Alkenoxiden. Bevorzugt sind Verbindungen mit ca. 5 bis 25 mol Ethylenoxid pro mol Alkylphenol. Kommerziell erhältliche Tenside diesen Typs sind z.B. Igepal® CO-630,Triton® X-45, X-114, X-100 und X 102, und die ®Arkopal-N-Marken der Clariant GmbH.

Kondensationsprodukte von aliphatischen Alkoholen mit ca. 1 bis ca. 25 mol Ethylenoxid.

Die Alkylkette der aliphatischen Alkohole kann linear oder verzweigt, primär oder sekundär sein, und enthält im allgemeinen ca. 8 bis ca. 22 Kohlenstoffatome. Besonders bevorzugt sind die Kondensationsprodukte von C₁₀-C₂₀-Alkoholen, mit ca. 2 bis ca. 18 mol Ethylenoxid pro mol Alkohol. Die Alkylkette kann gesättigt oder auch ungesättigt sein. Die Alkoholethoxilate können eine enge ("Narrow Range Ethoxilates") oder eine breite Homologenverteilung des Ethylenoxides ("Broad Range Ethoxylates") aufweisen. Beispiele von kommerziell erhältlichen nichtionischen Tensiden dieses Types sind Teritol® 15-S-9 (Kondensationsprodukt eines C₁₁-C₁₅ linearen sekundären Alkohols mit 9 mol Ethylenoxid), Tergitol® 24-L-NMW (Kondensationsprodukt eines C₁₂-C₁₄-linearen primären Alkohols mit 6 mol Ethylenoxid mit enger Molgewichtsverteilung). Ebenfalls unter diese Produktklasse fallen die Genapol®-Marken der Clariant GmbH.

Kondensationsprodukte von Ethylenoxid mit einer hydrophoben Basis, gebildet durch Kondensation von Propylenoxid mit Propylenglykol.

Der hydrophobe Teil dieser Verbindungen weist bevorzugt ein Molekulargewicht zwischen ca. 1500 und ca. 1800 auf. Die Anlagerung von Ethylenoxid an diesen hydrophoben Teil führt zu einer Verbesserung der Wasserlöslichkeit. Das Produkt ist flüssig bis zu einem Polyoxyethylengehalt von ca. 50 % des Gesamtgewichtes des Kondensationsproduktes, was einer Kondensation mit bis zu ca. 40 mol Ethylenoxid entspricht. Kommerziell erhältliche Beispiele dieser Produktklasse sind die Pluronic®-Marken der BASF und die ®Genapol PF-Marken der Clariant GmbH.

Kondensationsprodukt von Ethylenoxid mit einem Reaktionsprodukt von Propylenoxid und Ethylendiamin.

Die hydrophobe Einheit diese Verbindungen besteht aus dem Reaktionsprodukt von Ethylendiamin mit überschüssigem Propylenoxid und weist im allgemeinen ein Molekulargewicht von ca. 2500 bis ca. 3000 auf. An diese hydrophobe Einheit wird Ethylenoxid addiert, bis das Produkt einen Gehalt von ca. 40 bis ca. 80 Gew.-% Polyoxyethylen und ein Molekulargewicht von ca. 5000 bis ca. 11000 aufweist. Kommerziell erhältliche Beispiele dieser Verbindungsklasse sind die ®Tetronic-Marken der BASF und die ®Genapol PN-Marken der Clariant GmbH.

### Semipolare nichtionische Tenside

Diese spezielle Kategorie von nichtionischen Verbindungen umfaßt wasserlösliche Aminoxide, wasserlösliche Phosphinoxide und wasserlösliche Sulfoxide jeweils mit einem Alkylrest von ca. 1 0 bis ca. 18 Kohlenstoffatomen. Semipolare nichtionische Tenside sind auch Aminoxide der Formel

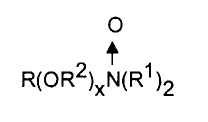

R ist hierbei eine Alkyl-, Hydroxyalkyl- oder Alkylphenolgruppe mit jeweils ca. 8 bis ca. 22 Kohlenstoffatomen, R² ist eine Alkylen- oder Hydroxyalkylengruppe mit ca. 2 bis 3 Kohlenstoffatomen oder Mischungen hiervon, jeder Rest R¹ ist eine Alkyl- oder Hydroxyalkylgruppe mit ca. 1 bis ca. 3 Kohlenstoffatomen oder eine Polyethylenoxidgruppe mit ca. 1 bis ca. 3 Ethylenoxideinheiten. Die R¹-Gruppen können miteinander über ein Sauerstoff- oder Stickstoffatom verbunden sein und somit einen Ring bilden. Aminoxide dieser Art sind besonders C₁₀-C₁₈-Alkyldimethylaminoxide und C₈-C₁₂-Alkoxiethyl-Dihydroxyethylaminoxide.

### Fettsäureamide

Fettsäureamide besitzen die Formel RCON(R¹)₂ , worin R eine Alkylgruppe mit ca. 7 bis ca. 21, bevorzugt ca. 9 bis ca. 17 Kohlenstoffatomen ist und jeder Rest R¹ Wasserstoff, C₁-C₄-Alkyl, C₁-C₄-Hydroxyalkyl oder (C₂H₄0)ₓH bedeutet, wobei x von ca. 1 bis ca. 3 variiert. Bevorzugt sind C₈-C₂₀-Amide, -monoethanolamide, -diethanolamide und -isopropanolamide.

Weitere geeignete nicht-ionische Tenside sind insbesondere Alkyl- und Alkenyloligoglycoside sowie Fettsäurepolyglykolester oder Fettaminpolyglykolester mit jeweils 8 bis 20, vorzugsweise 12 bis 18 C-Atomen im Fettalkylrest, alkoxylierte Triglycamide, Mischether oder Mischformale, Fettsäure-N-alkylglucamide, Proteinhydrolysate, Phosphinoxide oder Dialkylsulfoxide.

Typische Beispiele für amphotere bzw. zwitterionische Tenside sind Alkylbetaine, Alkylamidbetaine, Aminopropionate, Aminoglycinate, oder amphotere Imidazolinium-Verbindungen der Formel

R¹CONR⁴(CH₂)ₙ N^{⊕}R²R³CH₂Z X^{⊖}

worin R¹ C₈-C₂₂-Alkyl- oder -Alkenyl, R² Wasserstoff oder CH₂CO₂M, R³ CH₂CH₂OH oder CH₂CH₂OCH₂CH₂COOM, R⁴ Wasserstoff, CH₂CH₂OH oder CH₂CH₂COOM, Z CO₂M oder CH₂CO₂M, n 2 oder 3, bevorzugt 2, M Wasserstoff oder ein Kation wie Alkalimetall, Erdalkalimetall, Ammoniak oder Alkanolammonium bedeutet.

Bevorzugte amphotere Tenside dieser Formel sind Monocarboxylate und Dicarboxylate. Beispiele hierfür sind Cocoamphocarboxypropionat, Cocoamidocarboxypropionsäure, Cocoamphocarboxyglycinat (auch als Cocoamphodiacetat bezeichnet) und Cocoamphoacetat.

Weitere bevorzugte amphotere Tenside sind Alkyldimethylbetaine und Alkyldipolyethoxybetaine mit einem Alkylrest, der linear oder verzweigt sein kann, mit ca. 8 bis ca. 22 Kohlenstoffatomen, bevorzugt mit 8 bis 18 Kohlenstoffatomen und besonders bevorzugt mit ca. 12 bis ca. 18 Kohlenstoffatomen. Diese Verbindungen werden z.B. von der Clariant GmbH unter dem Handelsnamen ®Genagen LAB vermarktet.

Als kationische Tenside werden quartäre Ammoniumsalze vom Typ N^{⊕}R¹R²R³R⁴ X^{⊖} eingesetzt, worin R¹ C₈-C₂₄-Alkyl, bevorzugt C₁₀-C₁₈ n-Alkyl, R² C₁-C₄-Alkyl, bevorzugt Methyl, R³ die gleiche Bedeutung wie R¹ oder R² hat, R⁴ die gleiche Bedeutung wie R² hat oder Hydroxyethyl oder Hydroxypropyl bedeutet und X⁻ ein geeignetes Anion ist. Beispiele hierfür sind Distearyldimethylammoniumchlorid, Ditalgalkyldimethylammoniumchlorid, Ditalgalkylmethylhydroxypropylammoniumchlorid, Cetyltrimethylammoniumchlorid oder auch die entsprechenden Benzylderivate wie etwa Dodecyldimethylbenzylammoniumchlorid. Cyclische quartäre Ammoniumsalze, wie etwa Alkyl-Morpholinderivate können ebenfalls verwendet werden.

Darüberhinaus können neben den quartären Ammoniumverbindungen Imidazolinium-Verbindungen (1) und Imidazolinderivate (2) eingesetzt werden. 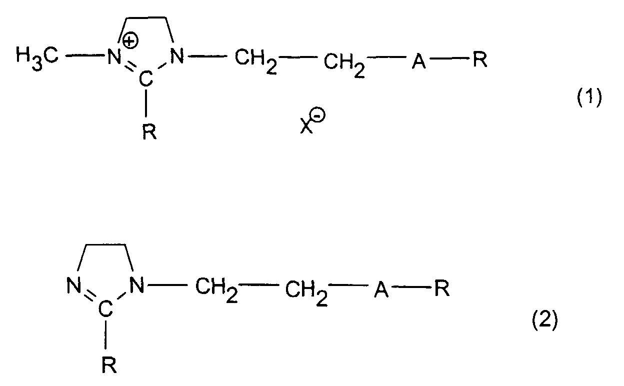 worin R C₈-C₂₄ n-, bzw. iso-Alkyl, bevorzugt C₁₀-C₁₈ n-Alkyl, X Bromid, Chlorid, Jodid, Methosulfat, A -NH-CO-, -CO-NH-, -O-CO-, -CO-O- ist.

Weitere geeignete kationische Tenside sind die sogenannten Esterquats. Es handelt sich hierbei um Umsetzungsprodukte von Alkanolaminen und Fettsäuren, die anschließend mit üblichen Alkylierungs- oder Hydroxyalkylierungsagenzien quaterniert werden.

Bevorzugt als Alkanolamine sind Verbindungen gemäß der Formel

NR¹R²R³

mit
- R¹ =: C₁-C₃ Hydroxyalkyl, bevorzugt Hydroxyethyl und
- R², R³ =: unabhängig voneinander R¹ oder C₁-C₃-Alkyl, bevorzugt Methyl.

Besonders bevorzugt sind Triethanolamin und Methyldiethanolamin.

Weitere besonders bevorzugte Ausgangsprodukte für Esterquats sind Aminoglycerinderivate, wie z.B. Dimethylaminopropandiol.

Alkylierungs- bzw. Hydroxyalkylierungsagenzien sind Alkylhalogenide, bevorzugt Methylchlorid, Dimethylsulfat, Ethylenoxid und Propylenoxid.

Beispiele für Esterquats sind Verbindungen der Formeln: 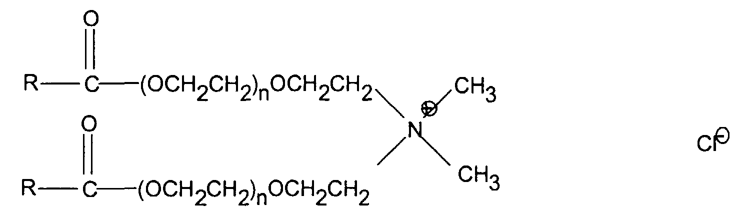 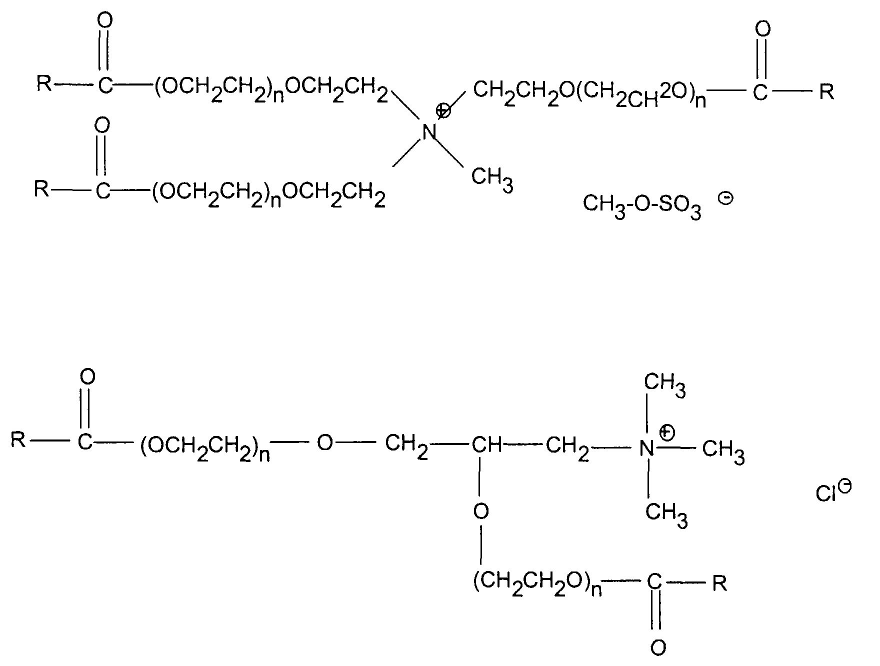 wobei RCO abgeleitet ist von C₈-C₂₄-Fettsäuren, die gesättigt oder ungesättigt sein können. Beispiele hierfür sind Capronsäure, Caprylsäure, hydrierte oder nicht oder nur teilweise hydrierte Talgfettsäuren, Stearinsäure, Ölsäure, Linolensäure, Behensäure, Palminstearinsäure, Myristinsäure und Elaidinsäure. n liegt im Bereich von 0 bis 10, vorzugsweise 0 bis 3, besonders bevorzugt 0 bis 1.

Die erfindungsgemäßen Formulierungen enthalten, je nach Anwendungszweck, neben den genannten Tensiden noch Wirkstoffe sowie die jeweils spezifischen Hilfs- und Zusatzstoffe. Bei den Wirkstoffen handelt es sich vorzugsweise um nichtflüchtige, flüssige Silicone. Dies kann entweder ein Polyalkylsiloxan, ein Polyarylsiloxan, ein Polyalkylarylsiloxan oder ein Polyethersiloxan-Copolymer sein und wird in einer Menge von ca. 0,1 % bis ca. 10,0 %, bevorzugt in einer Menge von ca. 0,5 % bis ca. 5,0 % eingesetzt.

Mischungen dieser Flüssigkeiten können ebenso verwendet werden und sind auch für bestimmte Anwendungen von Vorteil. Die dispergierten Siliconteilchen sollten unlöslich in der Shampoomatrix sein. Die wichtigsten nicht flüchtigen Polyalkylsiloxane, die eingesetzt werden können, sind z.B. Polydimethylsiloxane mit Viskositäten von ca. 5 bis ca. 600.000 Centistokes, vorzugsweise von 350 und ca. 100.000 Centistokes, bei 25°C. Ein geeignetes, im wesentlichen nicht flüchtiges Polyethersiloxan, ist z.B. ein mit Polypropylenoxid modifiziertes Dimethylpolysiloxan. Es können auch Addukte mit Ethylenoxid und/oder Propylenoxid eingesetzt werden.

Geeignete Silicone werden beispielsweise in US 2,826,551, US 3,945,500, US 4,364,837 und in GB 849,433 beschrieben.
Weiterhin kann Silicon-Gum erfindungsgemäß eingesetzt werden. Silicon-Gums sind in US 4,152,416 beschrieben, sowie in den Produktdatenblättern SE 30, SE 33, SE 54 und SE 76 der Firma General Electric. "Silicon Gum" bedeutet hochmolekulare Polydiorganosiloxane mit einer Molmasse von ca. 200.000 bis 1.000.000. Spezielle Beispiele sind Polydimethylsiloxan, (Polydimethylsiloxan) (Methylvinylsiloxan)-Copolymer, Poly(dimethylsiloxan)(Diphenyl)(Methylvinylsiloxyn)-Copolymer und ihre Mischungen, Mischungen von Siliconflüssigkeiten und Silicon-Gums sind ebenfalls geeignet.

Ein weiterer Wirkstoff, der in den Formulierungen enthalten sein kann, sind feste Antischuppenmittel. Hierfür kommen in Frage beispielsweise Schwefel, Selensulfid, Salicylsäure, Zink Pyrithion und 1-Hydroxy Pyridone, wie sie in US 4 185 106 beschrieben werden. Zink Pyrithion ist bevorzugt, besonders in Form von flachen, blättchenartigen Salzkristallen.

Geeignete und übliche Hilfsstoffe sind beispielsweise Überfettungsmittel, Verdicker, Feuchtigkeitsmittel, biogene Wirkstoffe, Filmbildner, Konditioniermittel, Konservierungsmittel, Parfüm oder Farbstoffe. Vorzugsweise handelt es sich hier bei den erfindungsgemäßen Formulierungen um Haarshampoos, die als weitere Hilfs- und Zusatzstoffe Emulgatoren wie etwa alkoxylierte Fettalkohole oder Sorbitanester enthalten können.

Als Überfettungsmittel können Substanzen wie beispielsweise polyoxyethylierte Lanolinderivate, Lecithinderivate und Fettsäurealkanolamide verwendet werden, wobei die letzteren gleichzeitig als Schaumstabilisatoren dienen.

Geeignete Verdickungsmittel sind beispielsweise Polysaccharide insbesondere Xanthan-Gum, Guar, Agar-Agar, Alginate und Tylosen, Carboxymethylcellulose und Hydroxyethylcellulose, ferner höhermolekulare Polyethylenglycolmono- und diester von Fettsäuren, Polyacrylate, Polyvinylalkohol und Polyvinylpyrrolidon sowie Elektrolyte wie Kochsalz und Ammoniumchlorid.

Unter biogenen Wirkstoffen sind beispielsweise Pflanzenextrakte und Vitaminkomplexe zu verstehen.

Gebräuchliche Filmbildner sind beispielsweise Chitosan, mikrokristallines Chitosan, quarterniertes Chitosan, Polyvinylpyrrolidon, Vinylpyrrolidon-Vinylacetat Copolymerisate, Polymere der Acrylsäurereihe, quaternäre Cellulose-Derivate und ähnliche Verbindungen.

Als Konservierungsmittel eignen sich beispielsweise Phenoxyethanol, Formaldehydlösung, Parabene, Pentadiol oder Sorbinsäure.

Als Farbstoffe können die für kosmetischen Zwecke geeigneten und zugelassenen Substanzen verwendet werden, wie sie beispielsweise in der Publikation "Kosmetische Färbemittel" der Farbstoffkommission der Deutschen Forschungsgemeinschaff Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung eingesetzt.

Der Gesamtanteil der Hilfs- und Zusatzstoffe kann 1 bis 50, vorzugsweise 5 bis 40 Gew.-%, bezogen auf die tensidhaltige Formulierung betragen.

### Beispiele

Im folgenden wird eine Reihe von speziellen Formulierungen gemäß der Erfindung angegeben. Alle Prozentangaben sind Gewichtsprozente.

Neben den in den jeweiligen Beispielen angegebenen Komponenten enthalten die Formulierungen noch Wasser sowie geringe Mengen Parfumöl, Konservierungsmittel, Farbstofflösung ad 100 Gew.-% der Formulierung.

| Beispiel 1 (Haarshampoo) | |
|---|---|
| ®Genapol LRO | 35,00 % |
| ®Hostapon KCG | 4,00 % |
| ®Merquat 550 | 1,00 % |
| ®Genagen CAB | 10,00 % |
| Genapol L-3 | 1,50 % |
| Genapol PEF | 4,00 % |
| Zinkchlorid | 0,06 % |

| Beispiel 2 (Duschbad) | |
|---|---|
| Hostapon SCID | 4,50 % |
| Genapol LRO | 35,00 % |
| ®Cetiol HE | 2,00 % |
| Genagen CAB | 10,00 % |
| Genapol PEF | 4,00 % |
| Zinkchlorid | 0,08 % |

| Beispiel 3 (Duschbad) | |
|---|---|
| Genapol LRO | 35,00 % |
| Genapol AMS | 12,00 % |
| Genagen CAB | 6,00 % |
| Cetiol HE | 2,00 % |
| Genapol L-3 | 0,50 % |
| Genapol PEF | 4,00 % |
| Zinkchlorid | 0,08 % |

| Beispiel 4 (Haarshampoo) | |
|---|---|
| Genapol LRO | 35,00 % |
| Genapol SBE | 4,00 % |
| Merquat 550 | 1,00 % |
| Genagen CAB | 6,00 % |
| Genapol L-3 | 1,00 % |
| Genapol PEF | 4,00 % |
| Zinkchlorid | 0,06 % |

| Beispiel 5 (Duschbad) | |
|---|---|
| Genapol LRO | 45,00 % |
| Hostapon KCG | 5,00 % |
| Cetiol HE | 2,00 % |
| Genagen CAB | 10,00 % |
| Genapol L-3 | 1,50 % |
| Genapol PEF | 4,00 % |
| Zinkchlorid | 0,06 % |

| Verzeichnis der eingesetzten Handelsprodukte | |
|---|---|
| ®Genagen CAB | Cocoamidopropyl Betaine, Clariant GmbH |
| ®Genapol LRO | Sodium Laureth Sulfate, Clariant GmbH |
| ®Genapol AMS | TEA-PEG-3 Cocoamide Sulfate, Clariant GmbH |
| ®Genapol L-3 | Laureth-3, Clariant GmbH |
| ®Genapol SBE | Disodium Laureth Sulfosuccinate, Clariant GmbH |
| ®Genapol PEF | Behenic Acid, Sodium Laureth Sulfate, Sodium Cocoyl Isethionat, Clariant GmbH |
| ®Hostapon KCG | Sodium Cocoyl Glutamat, Clariant GmbH |
| ®Hostapon SCID | Sodium Cocoyl Isethionate, Clariant GmbH |
| ®Cetiol HE | PEG-7 Glyceryl Cocoate, Henkel KGaA |
| ®Merquat 550 | Polyquaternium-7, Calgon Corporation |

## Patentansprüche

1. Tensidhaltige Formulierungen mit Perlglanzeffekt, enthaltend mindestens eine C₁₄-C₂₂-Fettsäure, ein Zn-, Mg-, Cu-, Al- oder Ti-Salz, ein Alkylethersulfat der Formel
R¹-O-(C₂H₄O)ₙ-SO₃^{⊕}X⁺
worin
R¹ C₁₂- bis C₁₈-Alkyl,
n eine Zahl von 2 bis 8,
X⁺ ein Alkalimetallion oder ein Ammoniumion bedeuten, und/oder
ein Acylisethionat der Formel
R²-CO-CH₂-CH₂-SO₃^{⊖}X⁺
worin
R² C₈- bis C₁₈-Alkyl,
X⁺ ein Alkalimetallion oder ein Ammoniumion bedeuten,
sowie Wasser und gegebenenfalls weitere Tenside und übliche Inhaltsstoffe.

2. Tensidhaltige Formulierungen nach Anspruch 1, dadurch gekennzeichnet, daß es sich um ein Perlglanzkonzentrat handelt.

3. Tensidhaltige Formulierung nach Anspruch 1, dadurch gekennzeichnet, daß es sich um eine gebrauchsfertige tensidhaltige Formulierung handelt.

4. Tensidhaltige Formulierung nach Anspruch 1, dadurch gekennzeichnet, daß es sich um ein Perlglanzkonzentrat handelt, das 5 bis 30 Gew.-% Fettsäure, 1 bis 50 Gew.-% Alkylethersulfat oder Acylisethionat oder deren Mischungen und 0,1 bis 20 Gew.-% Zn-, Mg-, Cu-, Al- oder Ti-Salz enthält.

5. Tensidhaltige Formulierung nach Anspruch 1, dadurch gekennzeichnet, daß es sich um eine gebrauchsfertige tensidhaltige Formulierung handelt, die 0,5 bis 50, vorzugsweise 2 bis 7 Gew.-% des Perlglanzkonzentrats gemäß Anspruch 2 enthält.

6. Tensidhaltige Formulierung nach Anspruch 1, dadurch gekennzeichnet, daß die Formulierung Behensäure als Fettsäure enthält.

7. Tensidhaltige Formulierung nach Anspruch 1, dadurch gekennzeichnet, daß die Formulierung ein Zink-Salz enthält.
